# EUROPEAN PATENT SPECIFICATION

(11) **EP 3 178 473 B1**
(45) Date of publication and mention of the grant of the patent: **13.09.2023**
(21) Application number: 15202468.3
(22) Date of filing: 23.12.2015
(51) Int. Cl.: A61K 31/167, A61K 9/48

(54) **ACID RESISTANT CAPSULE SHELL AND ITS PREPARATION PROCESS**
SÄUREBESTÄNDIGE KAPSELHÜLLE UND HERSTELLUNGSVERFAHREN DAFÜR
ENVELOPPE DE GÉLULE RÉSISTANT À L'ACIDE ET SON PROCÉDÉ DE PRÉPARATION

(30) Priority: 08.12.2015 US 201514963200
(43) Date of publication of application: 14.06.2017
(73) Proprietor: Dah Feng Capsule Industry Co., Ltd., Taichung 42756 (TW)
(72) Inventor: CHANG, Ruei Jan, 42756 Taichung (TW); LIN, Yi Huei, 42756 Taichung (TW); WU, Chieh Jen, 42756 Taichung (TW)
(74) Representative: dompatent von Kreisler Selting Werner - Partnerschaft von Patent- und Rechtsanwälten mbB

(56) References cited:
- EP-A1- 0 888 778
- EP-A1- 1 184 033
- EP-A1- 1 447 082
- EP-A1- 2 223 685
- WO-A1-03/084516
- WO-A1-2011/036601
- WO-A2-2008/119943

## Description

### BACKGROUND OF THE INVENTION

### 1. Field of the Invention

The present invention relates to an acid resistant capsule shell composition, an acid resistant capsule shell, and a process for preparing the acid resistant capsule shell.

### 2. Description of the Prior Art(s)

Capsules containing drugs and shells enclosing the drugs are widely applied in oral medication field. The capsules shells prevent the drugs being directly in contact with gustatory organ to cause nausea, being decomposed by saliva, and being deteriorated by moisture, air, or light. The capsules shells also delay the release time of the drugs.

The main component of the commercial capsules shell is gelatin; the commercial capsules are dissolved in gastric acid and release drugs at stomach. However, release of drugs such as nonsteroidal anti-inflammatory drugs at stomach may cause serious gastric side effects like damage of gastric mucosa, gastrorrhagia, or gastric perforation.

A conventional acid resistant capsule shell is developed to enclose the drugs, such that the drugs are released under intestinal condition instead of gastric condition to mitigate the gastric side effects of the drugs.

To prepare the general acid resistant capsule, one of the conventional processes prepares a general capsule shell, loads drugs in the general capsule shell, and coats an enteric film at the outer surface of the general capsule shell. The solubility of the enteric film is varied with pH value, i.e., the enteric film is soluble in alkaline condition but insoluble in acidic condition. Hence, the general acid resistant capsule can resist the gastric condition and release the drugs under intestinal condition. The component of enteric film is cellulose acetate phthalate (abbreviated as CAP), hydroxypropyl methylcellulose phthalate (abbreviated as HPMCP), hydroxypropyl methylcellulose acetate succinate (abbreviated as HPMP-AS), acrylic copolymers, or shellac. Since the components are required to be dissolved in organic solvents in the preparation, the general acid resistant capsule usually has undesired the organic solvent residues, and is also complicated to be prepared.

Another conventional process for preparing a general acid resistant capsule is double dipping method, which is refined from the conventional dip molding process. A pin of the double dipping method is dipped into a gelatin solution and an enteric coating solution subsequently, and then dried to form a general acid resistant capsule shell. Finally, drugs are loaded in the general acid resistant capsule shell to form the general acid resistant capsule. However, the component of the enteric coating solution is same with the component of the enteric film mentioned in previous paragraph; hence, the general acid resistant capsule prepared by the double dipping method also has organic solvent residues. Besides, the equipment of the double dipping method is more expensive than the equipment of the conventional dip molding process; therefore the cost of preparing the general acid resistant capsule may increase.

EP 1 184 033 A1 discloses film-forming compositions containing pectin, at least one additional film-forming polymer and a setting system for use in pharmaceutical, veterinary, food, cosmetic or other products like films for wrapping food, aspics or jellies, preferably for predosed formulations like soft or hard capsules.

As stated above, there is still a need to overcome the problems such as complex process, expensive equipments, and undesired organic solvent residues.

### SUMMARY OF THE INVENTION

The embodiments of the present invention are reflected in independent claims 1 and 7. The preferred embodiments of the present invention are reflected in dependent claims 2 to 6, 8 and 9.

The objective of the present invention is to modify the composition of an acid resistant capsule shell, such that the acid resistant capsule shell made from the composition can be prepared by the conventional dip molding process and equipment and is without organic solvent residues.

Another objective of the present invention is to render the acid resistant capsule shell an improved resistance to the gastric condition.

To achieve the foresaid objectives, the present invention provides an acid resistant capsule shell composition comprising a water-soluble enteric polymer, a water-soluble film forming polymer, a coagulant, and a gelling aid.

The water-soluble enteric polymer comprises hydrophobic functional groups and hydrophilic functional groups. A molecular weight of the water-soluble enteric polymer ranges from 20 kDa to 1000 kDa, inclusive. The water-soluble film forming polymer is selected from the group consisting of gelatin, pullulan, polyvinyl alcohol, modified starch, cellulose ester, and any combinations thereof. A molecular weight of the water-soluble film forming polymer ranges from 50 kDa to 815 kDa, inclusive. The coagulant comprises gellan gum or carrageen. A molecular weight of the coagulant ranges from 450 kDa to 550 kDa, inclusive. Based on the total weight of the water-soluble enteric polymer, the water-soluble film forming polymer, the coagulant, and the gelling aid, the weight percentage (wt%) of the water-soluble enteric polymer ranges from 5 wt% to 25 wt%, inclusive. The weight percentage of the water-soluble film forming polymer ranges from 71 wt% to 94.45 wt%, inclusive. The weight percentage of the coagulant ranges from 0.5 wt% to 3 wt%, inclusive. The weight percentage of the gelling aid ranges from 0.005 wt% to 1 wt%, inclusive.

The water-soluble enteric polymer is acidic resistance, i.e., the water-soluble enteric polymer is insoluble under gastric condition (pH value is about 1.2) and is soluble under intestinal condition (pH value is about 6.8). The water-soluble enteric polymer is different from the said water-soluble film forming polymer, that is, the water-soluble enteric polymer excludes gelatin, pullulan, polyvinyl alcohol, modified starch, and cellulose ester.

The acid resistant capsule shell composition can be treated as an initiator of an acid resistant capsule shell. The water-soluble enteric polymer, the water-soluble film forming polymer, the coagulant, and the gelling aid of the acid resistant capsule shell composition are all water-soluble. Hence, when the acid resistant capsule shell composition is applied to prepare the acid resistant capsule shell, the overall process for preparing the acid resistant capsule shell can be proceeded without organic solvent, and the problem of organic solvent residues can be prevented.

Preferably, the water-soluble enteric polymer comprises pectin, propylene glycol alginate (abbreviated as PGA), or xanthan gum; more preferably, the water-soluble enteric polymer comprises pectin or PGA; further preferably, the water-soluble enteric polymer comprises pectin.

Preferably, the molecular weight of the water-soluble enteric polymer ranges from 40 kDa to 400 kDa, inclusive; more preferably, the molecular weight of the water-soluble enteric polymer ranges from 50 kDa to 200 kDa, inclusive.

Preferably, a ratio of the hydrophobic functional groups to the hydrophilic functional groups of the water-soluble enteric polymer ranges from 30:70 to 40:60. When the ratio of the hydrophobic functional groups of the water-soluble enteric polymer is less than 30%, the acid resistant capsule shell prepared from the acid resistant capsule shell composition was more likely to be deformed due to excess absorption of water; therefore, drugs loaded in the acid resistant capsule shell are released in gastric condition .

Preferably, the hydrophobic functional groups of the water-soluble enteric polymer comprise methoxy group, propylene glycol, or their combination; the hydrophilic functional groups of the water-soluble enteric polymer comprise carboxyl group, amide group, or their combination; more preferably, the hydrophobic functional groups of the water-soluble enteric polymer comprise methoxy group; the hydrophilic functional groups of the water-soluble enteric polymer comprise carboxyl group.

Preferably, the modified starch comprises hydroxypropylated starch or hydroxyethylated starch.

Preferably, the cellulose ester comprises hydroxypropyl methylcellulose (abbreviated as HPMC), hydroxypropyl cellulose, methylcellulose, hydroxyethyl cellulose, or hydroxyethyl methylcellulose.

Preferably, a molecular weight of the water-soluble film forming polymer ranges from 50 kDa to 400 kDa, inclusive.

Preferably, the gelling aid is salt of single-valent cation or salt of divalent cation; more preferably, the gelling aid is salt of single-valent cation. The salt of single-valent cation is potassium chloride (abbreviated as KCl) or sodium chloride (abbreviated as NaCl). The salt of divalent cation is calcium chloride (abbreviated as CaCh) or magnesium chloride (abbreviated as MgCh).

The present invention further provides a process for preparing an acid resistant capsule shell. The process for preparing an acid resistant capsule shell comprises: dissolving said acid resistant capsule shell composition in deionized water to form a capsule shell solution; heating and then cooling the capsule shell solution to form a capsule shell stock solution; dipping a pin into the capsule shell stock solution then removing the pin to form a film-coated pin; and drying the film-coated pin to form the acid resistant capsule shell on the pin.

Preferably, the step of heating and then cooling the capsule shell solution to form the capsule shell stock solution comprises: heating the capsule shell solution at a temperature ranging from 65°C to 90°C, inclusive, and then cooling the capsule shell solution to form the capsule shell stock solution. More preferably, heating the capsule shell solution is at the temperature ranging from 75°C to 85°C, inclusive.

Preferably, the step of drying the film-coated pin to form the acid resistant capsule shell comprising: drying the film-coated pin at a temperature ranging from 20°C to 90°C to form the acid resistant capsule shell. More preferably, drying the film-coated pin is at the temperature ranging from 20°C to 80°C. Further preferably, drying the film-coated pin is at the temperature ranging from 20°C to 70°C.

Alternatively, drying the film-coated pin is at the temperature ranging from 70°C to 80°C.

Preferably, Step of heating and then cooling the capsule shell solution to form the capsule shell stock solution comprises: heating the capsule shell solution and then cooling the capsule shell solution at a temperature ranging from 50°C to 60°C,inclusive, to form the capsule shell stock solution.

Preferably, the temperature of the capsule shell stock solution need to be maintained between 50°C and 60°C, inclusive.

Preferably, the pH value of the capsule shell stock solution ranges from 4 to 6, inclusive.

The present invention further provides an acid resistant capsule shell. The acid resistant capsule shell comprises a water-soluble enteric polymer, a water-soluble film forming polymer, a coagulant, and a moisture. The water-soluble enteric polymer has hydrophobic functional groups and hydrophilic functional groups. A molecular weight of the water-soluble enteric polymer ranges from 20 kDa to 1000 kDa, inclusive. The water-soluble film forming polymer selected from the group consisting of gelatin, pullulan, polyvinyl alcohol, modified starch, cellulose ester, and any combinations thereof, and a molecular weight of the water-soluble film forming polymer ranging from 50 kDa to 815 kDa, inclusive. The coagulant comprises gellan gum or carrageen. A molecular weight of the coagulant ranges from 450 kDa to 550 kDa, inclusive. Based on the total weight of the acid resistant capsule shell, the weight percentage of the water-soluble enteric polymer ranges from 5 wt% to 25 wt%, inclusive. The weight percentage of the water-soluble film forming polymer ranges from 65 wt% to 90.5 wt%, inclusive. The weight percentage of the coagulant ranges from 0.5 wt% to 3 wt%, inclusive. The weight percentage of the moisture ranges from 4 wt% to 7 wt%, inclusive.

Preferably, the water-soluble enteric polymer comprises pectin, PGA, or xanthan gum; more preferably, the water-soluble enteric polymer comprises pectin or PGA; further preferably, the water-soluble enteric polymer comprises pectin.

Preferably, the molecular weight of the water-soluble enteric polymer ranges from 40 kDa to 400 kDa, inclusive; more preferably, the molecular weight of the water-soluble enteric polymer ranges from 50 kDa to 200 kDa, inclusive.

Preferably, a ratio of the hydrophobic functional groups to the hydrophilic functional groups of the water-soluble enteric polymer ranges from 30:70 to 40:60.

Preferably, the hydrophobic functional groups of the water-soluble enteric polymer comprise methoxy group, propylene glycol, or their combination; the hydrophilic functional groups of the water-soluble enteric polymer comprise carboxyl group, amino group, or their combination; more preferably, the hydrophobic functional groups of the water-soluble enteric polymer comprise methoxy group; the hydrophilic functional groups of the water-soluble enteric polymer comprise carboxyl group.

Preferably, the modified starch comprises hydroxypropylated starch or hydroxyethylated starch.

Preferably, the cellulose ester comprises HPMC, hydroxypropyl cellulose, methylcellulose, hydroxyethyl cellulose, or hydroxyethyl methylcellulose.

Preferably, a molecular weight of the water-soluble film forming polymer ranges from 50 kDa to 400 kDa, inclusive.

Other objectives, advantages and novel features of the invention will become more apparent from the following detailed description when taken in conjunction with the accompanying drawings.

### DETAILED DESCRIPTION OF INVENTION TECHNOLOGY

1. The composition of acid resistant capsule shell includes :
   1. a film forming polymer selected from the group consisting of HPMC, cellulose ester, gelatin, pullulan and so on.
   2. 3-30% pH sensitive materials (pectin, xanthan gum, Propylene Glycol Alginate)
   3. 0.3-3% gelling agents (gellan gum, carrageenan , alginate, agar, konjac gum, locust beam gum, hypromellose, hydroxyzine hydrochloric)
2. The acid resistant capsule is pH sensitive, it does not dissolve in acidic condition, but dissolves very quickly in neutral or alkaline condition. So it can protect the content from the erosion of stomach acid and release the content under intestinal condition.
3. If the natural water- soluble biopolymer contains -COOH functional groups, it won't dissolve in acid condition and can dissolve quickly in neutral or alkaline condition.
4. However, if the water soluble biopolymer only contains carboxyl groups, the acid resistant capsule shell prepared from this material is more likely to be deformed due to excess absorption of water and swell very easily, then the content is released.
5. The soluble biopolymer must contain the hydrophobic functional groups, to keep the material from swell or absorb water at acidic condition. the hydrophobic functional groups of the water-soluble enteric polymer comprise methoxyl group, methyl group, ethyl group and any hydrophobic functional groups...
6. The suitable water-soluble enteric polymer comprises hydrophobic functional groups (methoxyl group, methyl group , ethyl group and any hydrophobic functional groups)and hydrophilic functional groups(carboxyl group, amide group, hydroxyl group... )
7. The acid resistant capsule shell formulation, wherein a ratio of the hydrophobic functional groups to the hydrophilic functional groups of the water-soluble enteric polymer ranges from 30:70 to 40:60.
8. The water-soluble enteric polymer including pectin, propylene glycol alginate, or xanthan gum...et al. can formulate to acid resistant capsule shell.
9. Our processes coagulant including gellan gum, carrageenan gum, alginate, agar, konjac gum, locust beam gum....
10. The gelling aid includes: salt of single-valent cation (k Na) or salt of divalent cation(Ca Mg).
11. The salt of divalent cation is not necessary in the acid resistant capsule formulation. Thus, the capsule shell is not cracked in our manufacture process even at high drying temperature.

### DETAILED DESCRIPTION OF THE PREFERRED EMBODIMENTS

### Embodiment 1

Basic embodiment, comprising acid-resistant material: pectin, coagulant: gellan gum, gelling aid: KC1, film forming polymer material: HPMC.

The process for preparing an acid resistant capsule shell was described as follows.

First, an acid resistant capsule shell composition was provided. The acid resistant capsule shell composition comprised a water-soluble enteric polymer, a water-soluble film forming polymer, a coagulant, and a gelling aid. The water-soluble enteric polymer has acidic resistance, i.e., the water-soluble enteric polymer is insoluble under gastric condition (about pH 1.2) and is soluble under intestinal condition (about pH 6.8). In the present embodiment, the water-soluble enteric polymer was pectin. The molecular weight of pectin ranged from 40 kDa to 400 kDa. Pectin comprised hydrophobic functional groups, methoxy group, and hydrophilic functional groups, carboxyl group. The ratio of the hydrophobic functional groups to the hydrophilic functional groups of pectin was 30:70 and the ratio of the hydrophobic functional groups to the hydrophilic functional groups of pectin was measured according to the instruction of The United States Pharmacopeial Convention <29> (abbreviated as USP<29>). The water-soluble film forming polymer was HPMC. The molecular weight of HPMC was about 80 kDa. The coagulant was gellan gum. The molecular weight of gellan gum was about 500 kDa. The gelling aid was KCl. The usages of the water-soluble enteric polymer, the water-soluble film forming polymer, the coagulant, and the gelling aid of the acid resistant capsule shell composition were listed in Table 1.

998.05 grams of the acid resistant capsule shell composition was dissolved in 6.1 kilograms of deionized water to form a capsule shell solution. The capsule shell solution was stirred at 80°C to make the acid resistant capsule shell composition dissolve completely, and then the capsule shell solution was cooled to 55°C to form a capsule shell stock solution. The pH value of the capsule shell stock solution was 4.5.

The capsule shell stock solution was poured into a glue pool and maintained at 50°C to 55°C. A pin was dipped into the capsule shell stock solution and then removed to form a film-coated pin. Finally, the film-coated pin was dried at 80°C to form a capsule shell on the pin. The acid resistant capsule shell comprised HPMC, gellan gum, pectin, and moisture. The weight percentages of said component of the acid resistant capsule shell were listed in Table 1.

**Table 1: the usages and weight percentages of said component of the acid resistant capsule shell**

| Embodiment | acid resistant capsule shell composition | | | acid resistant capsule shell | |
|---|---|---|---|---|---|
| | Component | Usage (g) | wt% | Component | wt% |
| Embodiment 1 | HPMC | 890 | 89.17 | HPMC | 84.00 |
| | gellan gum | 8 | 0.80 | gellan gum | 0.70 |
| | Pectin | 100 | 10.02 | pectin | 9.40 |
| | KCl | 0.05 | 0.01 | moisture | 5.90 |
| | Total | 998.05 | 100.00 | Total | 100.00 |

| | Component | Usage (g) | wt% | Component | wt% |
|---|---|---|---|---|---|
| Embodiment 2 | Pullulan | 890 | 89.17 | pullulan | 84.00 |
| | gellan gum | 8 | 0.80 | gellan gum | 0.70 |
| | Pectin | 100 | 10.02 | pectin | 9.40 |
| | KCl | 0.05 | 0.01 | moisture | 5.90 |
| | Total | 998.05 | 100.00 | Total | 100.00 |

| | Component | Usage (g) | wt% | Component | wt% |
|---|---|---|---|---|---|
| Embodiment 3 | HPMC | 910 | 91.18 | HPMC | 86.00 |
| | gellan gum | 8 | 0.80 | gellan gum | 0.75 |
| | PGA | 80 | 8.02 | PGA | 7.50 |
| | KCl | 0.05 | 0.01 | moisture | 5.75 |
| | Total | 998.05 | 100.00% | Total | 100.00% |

| | Component | Usage (g) | wt% | Component | wt% |
|---|---|---|---|---|---|
| Embodiment 4 | HPMC | 840 | 84.16 | HPMC | 79.25 |
| | gellan gum | 8 | 0.80 | gellan gum | 0.75 |
| | Pectin | 150 | 15.03 | pectin | 14.1 |
| | KCl | 0.05 | 0.01 | moisture | 5.9 |
| | Total | 998.05 | 100.00 | Total | 100 |

| | Component | Usage (g) | wt% | Component | wt% |
|---|---|---|---|---|---|
| Comparative Embodiment 1 | HPMC | 840 | 84.16 | HPMC | 84.00 |
| | gellan gum | 8 | 0.80 | gellan gum | 0.70 |
| | Pectin | 150 | 15.03 | pectin | 9.40 |
| | KCl | 0.05 | 0.01 | moisture | 5.90 |
| | Total | 998.05 | 100.00 | Total | 100.00 |

| | Component | Usage (g) | wt% | Component | wt% |
|---|---|---|---|---|---|
| Comparative Embodiment 2 | HPMC | 890 | 89.17 | HPMC | 83.95 |
| | gellan gum | 8 | 0.80 | gellan gum | 0.75 |
| | Pectin | 100 | 10.02 | pectin | 9.4 |
| | CaCl₂ | 0.05 | 0.01 | moisture | 5.9 |
| | Total | 998.05 | 100.00 | Total | 100 |

| | Component | Usage (g) | wt% | Component | wt% |
|---|---|---|---|---|---|
| Comparative Embodiment 3 | HPMC | 840 | 84.16 | HPMC | 84.00 |
| | gellan gum | 8 | 0.80 | gellan gum | 0.70 |
| | Pectin | 150 | 15.03 | pectin | 9.40 |
| | KCl | 0.05 | 0.01 | moisture | 5.90 |
| | Total | 998.05 | 100.00 | Total | 100.00 |

| | Component | Usage (g) | wt% | Component | wt% |
|---|---|---|---|---|---|
| New Embodiment | HPMC | 840 | 84.16 | HPMC | 79.19 |
| | gellan gum | 8 | 0.80 | gellan gum | 0.75 |
| | Xanthan gum | 150 | 15.03 | Xanthan gum | 14.91 |
| | KCl | 0.05 | 0.01 | moisture | 5.90 |
| | Total | 998.05 | 100.00 | Total | 100.00 |

### Embodiment 2

Comprising, acid-resistant material: pectin, coagulant: gellan gum, gelling aid : KC1, film forming polymer material: Pullulan.

A process for preparing an acid resistant capsule shell of the instant embodiment was similar with the Embodiment 1. The difference between these two embodiments was that the water-soluble film forming polymer of Embodiment 2 was pullulan. The molecular weight of pullulan was about 805 kDa. The usages of the water-soluble enteric polymer, the water-soluble film forming polymer, the coagulant, and the gelling aid of the acid resistant capsule shell composition were listed in Table 1. The weight percentages of components of the acid resistant capsule shell were listed in Table 1.

### Embodiment 3

Comprising, acid-resistant material: PGA, coagulant: gellan gum, gelling aid : KC1, film forming polymer material: HPMC.

A process for preparing an acid resistant capsule shell of the instant embodiment was similar with the Embodiment 1. The difference between these two embodiments was that the water-soluble enteric polymer was PGA. The molecular weight of PGA was about 240 kDa. PGA comprised hydrophobic functional groups, propylene glycol group, and hydrophilic functional groups, carboxyl group. The ratio of the hydrophobic functional groups to the hydrophilic functional groups of PGA was 35:65. The water-soluble film forming polymer was HPMC. The molecular weight of HPMC was about 130 kDa. The usages of the water-soluble enteric polymer, the water-soluble film forming polymer, the coagulant, and the gelling aid of the acid resistant capsule shell composition were listed in Table 1. The weight percentages of components of the acid resistant capsule shell were listed in Table 1.

### Embodiment 4

Comprising, acid-resistant material: pectin, coagulant: gellan gum, gelling aid : KC1, film forming polymer material: HPMC.

A process for preparing an acid resistant capsule shell of the instant embodiment was similar with the Embodiment 1. The difference between these two embodiments was that the ratio of the hydrophobic functional groups to the hydrophilic functional groups of pectin was 40:60. The film-coated pin was dried at 30°C to form the acid resistant capsule shell. The usages of the water-soluble enteric polymer, the water-soluble film forming polymer, the coagulant, and the gelling aid of the acid resistant capsule shell composition were listed in Table 1. The weight percentages of components of the acid resistant capsule shell were listed in Table 1.

### Comparative Embodiment 1

Comprising, acid-resistant material: pectin, coagulant: gellan gum, gelling aid : KC1, film forming polymer material: HPMC.

This comparative embodiment provided a process for preparing an acid resistant capsule shell which was similar with the Embodiment 1. The difference between the Comparative Embodiment 1 and the Embodiment 1 was that the ratio of the hydrophobic functional groups to the hydrophilic functional groups of pectin was 28:72. The usages of the water-soluble enteric polymer, the water-soluble film forming polymer, the coagulant, and the gelling aid of the acid resistant capsule shell composition were listed in Table 1. The weight percentages of components of the acid resistant capsule shell were listed in Table 1.

### Comparative Embodiment 2

Comprising, acid-resistant material: pectin, coagulant: gellan gum, gelling aid : CaCl2, film forming polymer material: HPMC.

This comparative embodiment provided a process for preparing an acid resistant capsule shell which was similar with the Embodiment 1. The difference between the Comparative Embodiment 2 and the Embodiment 1 was that the gelling aid was calcium chloride. The capsule shell solution stirred at 90°C to make the acid resistant capsule shell composition dissolve completely. The film-coated pin was dried at 70°C to form the acid resistant capsule shell. However, the surface of the acid resistant capsule shell formed some cracks after drying. The usages of the water-soluble enteric polymer, the water-soluble film forming polymer, the coagulant, and the gelling aid of the acid resistant capsule shell composition were listed in Table 1. The weight percentages of components of the acid resistant capsule shell were listed in Table 1

### Comparative Embodiment 3

Comprising, acid-resistant material: pectin, coagulant: gellan gum, gelling aid : KC1, film forming polymer material: HPMC.

This comparative embodiment provided a process for preparing an acid resistant capsule shell which was similar with the Embodiment 1. The difference between the Comparative Embodiment 3 and the Embodiment 1 was that the ratio of the hydrophobic functional groups to the hydrophilic functional groups of pectin was 72:28. The usages of the water-soluble enteric polymer, the water-soluble film forming polymer, the coagulant, and the gelling aid of the acid resistant capsule shell composition were listed in Table 1. The weight percentages of components of the acid resistant capsule shell were listed in Table 1.

Comprising, acid-resistant material: Xanthan gum, coagulant: gellan gum, gelling aid : KCl, film forming polymer material: HPMC.

This comparative embodiment provided a process for preparing an acid resistant capsule shell which was similar with the Embodiment 1. The difference between these two embodiments was that the water-soluble enteric polymer was xanthan gum, It is composed of pentasaccharide repeat units, comprising glucose, mannose, and glucuronic acid in the molar ratio 2:2:1, comprised hydrophobic functional groups, methyl group, and hydrophilic functional groups, carboxyl group. The water-soluble film forming polymer, the coagulant, and the gelling aid of the acid resistant capsule shell composition were listed in Table 1. The weight percentages of components of the acid resistant capsule shell were listed in Table 1.

In another embodiment, the formula may not only be used to fabricate the capsule, but also to form the band that encapsulates the capsule. The band may be sued as a marker or a fastener to fasten close a bifurcated capsule.

The gel solution which prepared from the embodiment 4 can also be used as banding solution. The banding has two objectives: to prevent the possible liquid leak during storage and to improve acid resistance of the acid resistant capsule. By dissolving the capsules which prepared from the embodiment 4 in hot water can be used as banding solution. The viscosity of banding solution was about 1200cps.

The banding operation was performed at the speed of 80 000capsules /hour with drying air conditions: 20°C/24%RH.

### Experimental Embodiment

Acetaminophen (solid powder) was respectively loaded into the acid resistant capsule shell prepared from the Embodiment 1 to the Embodiment 4 and the Comparative Embodiment 1 to the Comparative Embodiment 3 to form an acid resistant capsule of said embodiments and comparative embodiments, and then the acid resistant capsules were proceeded with an in-vitro dissolution test according to the instruction of USP<711>.

In the first stage of the in-vitro dissolution test, the acid resistant capsule was put into a simulated gastric acid solution for 2 hours, and then the concentration of acetaminophen of the simulated gastric acid solution in the first stage of the in-vitro dissolution test was measured. The pH value of the simulated gastric acid solution was 1.2, and the temperature of the simulated gastric acid solution was 37°C. According to the instruction of USP<711>, the dissolution rate in the first stage of the in-vitro dissolution test was calculated based on the weight of acetaminophen and the concentration of acetaminophen of the simulated gastric acid solution in the first stage of the in-vitro dissolution test.

The second stage of the in-vitro dissolution test was proceeded after the first stage of the in-vitro dissolution test. In the second stage of the in-vitro dissolution test, the pH value of the simulated gastric acid solution was adjusted to 6.8 within 5 minutes to form a simulated intestinal solution. The acid resistant capsule was kept in the simulated intestinal solution 45 minutes, and then the concentration of acetaminophen of the simulated intestinal solution in the second stage of the in-vitro dissolution test was measured. According to the instruction of USP<711>, the dissolution rate in the second stage of the in-vitro dissolution test was calculated based on the weight of acetaminophen and the concentration of acetaminophen of the simulated intestinal solution in the second stage of the in-vitro dissolution test. The dissolution rate in the first stage of the in-vitro dissolution test and the dissolution rate in the second stage of the in-vitro dissolution test of the Embodiment 1 to the Embodiment 4, the Comparative Embodiment 1, and the Comparative Embodiment 3 were listed in Table 2.

**Table 2: the dissolution rate in the first stage of the in-vitro dissolution test and the dissolution rate in the second stage of the in-vitro dissolution test of the Embodiment 1 to the Embodiment 4, the Comparative Embodiment 1, and the Comparative Embodiment 3**

| Embodiment | The dissolution rate in the first stage (%) | The dissolution rate in the second stage (%) |
|---|---|---|
| Embodiment 1 | 9.6 | 85.7 |
| Embodiment 2 | 9.5 | 84.5 |
| Embodiment 3 | 15.4 | 60.5 |
| Embodiment 4 | 5.9 | 87.7 |
| Comparative Embodiment 1 | 35 | 50 |
| Comparative Embodiment 3 | 38 | 80 |
| New comparative Embodiment 2 | 2.2 | 75 |

With reference to Table 2, the shape of the acid resistant capsule prepared from the Comparative Embodiment 1 was deformed and the dissolution rate in the first stage of the in-vitro dissolution test of the acid resistant capsule prepared from the Comparative Embodiment 1 was 35% because the ratio of the hydrophobic functional groups to the hydrophilic functional groups of pectin was 28:72. When the ratio of the hydrophilic functional groups of pectin was too high, the acid resistant capsule was more likely to be deformed due to excess absorption of water; therefore, acetaminophen was released in gastric condition. Compared to the acid resistant capsule prepared from the Embodiments 1 to 4, the dissolution rates in the first stage of the in-vitro dissolution test of the acid resistant capsule prepared from the Embodiments 1 to 4 were less than 16%, and the shapes of the acid resistant capsule prepared from the Embodiments 1 to 4 were intact. Furthermore, the dissolution rates in the second stage of the in-vitro dissolution test of the acid resistant capsule prepared from the Embodiments 1 to 4 were greater than 60%, indicating that the acid resistant capsule prepared from the Embodiments 1 to 4 have excellent acidic resistance.

According to foresaid preparation of the Comparative Embodiment 2, which adopted the excess calcium chloride as the gelling aid, the acid resistant capsule shell of the Comparative Embodiment 2 was ruptured and became unusable after drying at 70°C. The acid resistant capsule of Comparative Embodiment 2 could not be examined by the first and second stages of the in-vitro dissolution test. If the gelling aid is calcium chloride or other divalent cations, the film-coated pin must be dried at less than 60°C to prevent the rupture of the acid resistant capsule shell. However, drying the film-coated pin at a low temperature prolongs the manufacturing time. Compared to the acid resistant capsule prepared from the Embodiment 1, the dissolution rate in the first stage of the in-vitro dissolution test of the acid resistant capsule prepared from the Embodiment 1 was less than 10% and the shape of the acid resistant capsule prepared from the Embodiment 1 was intact. That is to say, the film-coated pin of acid resistant capsule shell of the Embodiment 1 could be dried at a higher temperature than that of the Comparative Example 2, and thus the acid resistant capsule shell of Embodiment 1 made from said composition is more beneficial for rapid production.

The dissolution rate in the first stage of the in-vitro dissolution test of the acid resistant capsule prepared from the Comparative Embodiment 3 was 38% because the ratio of the hydrophobic functional groups to the hydrophilic functional groups of pectin was 72:28. When the ratio of the hydrophobic functional groups of pectin was too high, the acid resistant capsule was more likely to be dissolved in simulated gastric acid solution; therefore, acetaminophen was released.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. An acid resistant capsule shell composition comprising:
a water-soluble enteric polymer having hydrophobic functional groups and hydrophilic functional groups;
a water-soluble film forming polymer selected from the group consisting of gelatin, pullulan, polyvinyl alcohol, modified starch, cellulose ester, and any combinations thereof;
a coagulant selected from the group consisting of gellan gum, carrageen, alginate, agar, konjac gum and locust beam gum; and
a gelling aid which is a salt of single-valent cation; and
wherein based on the total weight of the water-soluble enteric polymer, the water-soluble film forming polymer, the coagulant, and the gelling aid, the weight percentage of the water-soluble enteric polymer ranges from 3 wt% to 25 wt%, the weight percentage of the water-soluble film forming polymer ranges from 71 wt% to 96.5 wt%, the weight percentage of the coagulant ranges from 0.5 wt% to 3 wt%, and the weight percentage of the gelling aid ranges from 0.005 wt% to 1 wt%;
wherein a ratio of the hydrophobic functional groups to the hydrophilic functional groups of the water-soluble enteric polymer ranges from 30:70 to 40:60; and
wherein the water-soluble enteric polymer has a molecular weight of from 20 kDa to 1000 kDa, inclusive; the water-soluble film forming polymer has a molecular weight of from 50 kDa to 815 kDa, inclusive; and the coagulant has a molecular weight of from 450 kDa to 550 kDa, inclusive.

2. The acid resistant capsule shell composition as claimed in claim 1, wherein the hydrophobic functional groups of the water-soluble enteric polymer are selected from methoxy group, propylene glycol, methyl group, ethyl group or their combination, preferably methoxy group.

3. The acid resistant capsule shell composition as claimed in one of claims 1 to 2, wherein the hydrophilic functional groups of the water-soluble enteric polymer comprise carboxyl group, amide group, hydroxyl group or their combination.

4. The acid resistant capsule shell composition as claimed in one of claims 1 to 3, wherein the hydrophilic functional groups of the water-soluble enteric polymer comprise carboxyl group.

5. The acid resistant capsule shell composition as claimed in one of claims 1 to 4, wherein the water-soluble enteric polymer comprises at least one selected of the following: pectin, propylene glycol alginate, and xanthan gum.

6. The acid resistant capsule shell composition as claimed in one of claims 1 to 5, wherein the water-soluble enteric polymer comprises pectin or propylene glycol alginate.

7. A process for preparing an acid resistant capsule shell comprising:
dissolving the acid resistant capsule shell composition as claimed in one of claims 1 to 6 in deionized water to form a capsule shell solution;
heating and then cooling the capsule shell solution to form a capsule shell stock solution;
dipping a pin into the capsule shell stock solution then removing the pin to form a film-coated pin; and
drying the film-coated pin to form the acid resistant capsule shell.

8. The process for preparing an acid resistant capsule shell as claimed in claim 7, wherein the step of heating and then cooling the capsule shell solution to form the capsule shell stock solution, further comprises heating the capsule shell solution at a temperature ranging from 65°C to 90°C and then cooling the capsule shell solution to form the capsule shell stock solution.

9. The process for preparing an acid resistant capsule shell as claimed in claim 7 or 8, wherein the step of drying the film-coated pin to form the acid resistant capsule shell, further comprises drying the film-coated pin at a temperature ranging from 20°C to 90°C to form the acid resistant capsule shell.

## Patentansprüche

1. Säurebeständige Kapselhüllenzusammensetzung, umfassend:
ein wasserlösliches magensaftresistentes Polymer, das hydrophobe funktionelle Gruppen und hydrophile funktionelle Gruppen aufweist;
ein wasserlösliches filmbildendes Polymer, das aus der Gruppe ausgewählt ist, die aus Gelatine, Pullulan, Polyvinylalkohol, modifizierter Stärke, Celluloseester und irgendwelchen Kombinationen davon besteht;
ein Koagulans, das aus der Gruppe ausgewählt ist, die aus Gellan, Carrageen, Alginat, Agar, Konjakmehl und Johannisbrotkernmehl besteht; und
ein Gelierhilfsmittel, das ein Salz eines einwertigen Kations ist;
wobei, bezogen auf das Gesamtgewicht des wasserlöslichen magensaftresistenten Polymers, des wasserlöslichen filmbildenden Polymers, des Koagulans und des Gelierhilfsmittels, der prozentuale Gewichtsanteil des wasserlöslichen magensaftresistenten Polymers im Bereich von 3 Gew.-% bis 25 Gew.-% liegt, der prozentuale Gewichtsanteil des wasserlöslichen filmbildenden Polymers im Bereich von 71 Gew.-% bis 96,5 Gew.-% liegt, der prozentuale Gewichtsanteil des Koagulans im Bereich von 0,5 Gew.-% bis 3 Gew.-% liegt und der prozentuale Gewichtsanteil des Gelierhilfsmittels im Bereich von 0,005 Gew.-% bis 1 Gew.-% liegt;
wobei das Verhältnis der hydrophoben funktionellen Gruppen zu den hydrophilen funktionellen Gruppen des wasserlöslichen magensaftresistenten Polymers im Bereich von 30:70 bis 40:60 liegt; und
wobei das wasserlösliche magensaftresistente Polymer ein Molekulargewicht von 20 kDa bis 1000 kDa einschließlich aufweist; das wasserlösliche filmbildende Polymer ein Molekulargewicht von 50 kDa bis 815 kDa einschließlich aufweist; und das Koagulans ein Molekulargewicht von 450 kDa bis 550 kDa einschließlich aufweist.

2. Säurebeständige Kapselhüllenzusammensetzung gemäß Anspruch 1, wobei die hydrophoben funktionellen Gruppen des wasserlöslichen magensaftresistenten Polymers aus einer Methoxygruppe, Propylenglycol, Methylgruppe, Ethylgruppe oder einer Kombination davon, vorzugsweise Methoxygruppe, ausgewählt sind.

3. Säurebeständige Kapselhüllenzusammensetzung gemäß einem der Ansprüche 1 bis 2, wobei die hydrophilen funktionellen Gruppen des wasserlöslichen magensaftresistenten Polymers eine Carboxygruppe, Amidgruppe, Hydroxygruppe oder eine Kombination davon umfassen.

4. Säurebeständige Kapselhüllenzusammensetzung gemäß einem der Ansprüche 1 bis 3, wobei die hydrophilen funktionellen Gruppen des wasserlöslichen magensaftresistenten Polymers eine Carboxygruppe umfassen.

5. Säurebeständige Kapselhüllenzusammensetzung gemäß einem der Ansprüche 1 bis 4, wobei das wasserlösliche magensaftresistente Polymer wenigstens eines umfasst, das aus den folgenden ausgewählt ist: Pektin, Propylenglycolalginat und Xanthan.

6. Säurebeständige Kapselhüllenzusammensetzung gemäß einem der Ansprüche 1 bis 5, wobei das wasserlösliche magensaftresistente Polymer Pektin oder Propylenglycolalginat umfasst.

7. Verfahren zur Herstellung einer säurebeständigen Kapselhülle, umfassend:
das Auflösen der säurebeständigen Kapselhüllenzusammensetzung gemäß einem der Ansprüche 1 bis 6 in deionisiertem Wasser unter Bildung einer Kapselhüllenlösung;
das Erhitzen und dann Abkühlen der Kapselhüllenlösung unter Bildung einer Kapselhüllenstammlösung;
das Eintauchen eines Stifts in die Kapselhüllenstammlösung, dann Herausnehmen des Stifts unter Bildung eines filmbeschichteten Stifts; und
das Trocknen des filmbeschichteten Stifts unter Bildung der säurebeständigen Kapselhülle.

8. Verfahren zur Herstellung einer säurebeständigen Kapselhülle gemäß Anspruch 7, wobei der Schritt des Erhitzens und dann Abkühlens der Kapselhüllenlösung unter Bildung der Kapselhüllenstammlösung weiterhin das Erhitzen der Kapselhüllenlösung auf eine Temperatur im Bereich von 65°C bis 90°C und dann das Abkühlen der Kapselhüllenlösung unter Bildung der Kapselhüllenstammlösung umfasst.

9. Verfahren zur Herstellung einer säurebeständigen Kapselhülle gemäß Anspruch 7 oder 8, wobei der Schritt des Trocknens des filmbeschichteten Stifts unter Bildung der säurebeständigen Kapselhülle weiterhin das Trocknen des filmbeschichteten Stifts bei einer Temperatur im Bereich von 20°C bis 90°C unter Bildung der säurebeständigen Kapselhülle umfasst.

## Revendications

1. Composition d'enveloppe de capsule résistante aux acides, comprenant :
un polymère entérique soluble dans l'eau présentant des groupes fonctionnels hydrophobes, et des groupes fonctionnels hydrophiles,
un polymère filmogène soluble dans l'eau choisi dans le groupe consistant en gélatine, pullulan, alcool polyvinylique, amidon modifié, ester cellulosique, et des combinaisons quelconques de ceux-ci,
un coagulant choisi dans le groupe consistant en gomme gellane, carraghénane, alginate, agar, konjac, et gomme de caroube, et
un auxiliaire de gélification qui est un sel d'un cation monovalent, et
dans laquelle, par rapport au poids total du polymère entérique soluble dans l'eau, du polymère filmogène soluble dans l'eau, du coagulant et de l'auxiliaire de gélification, le pourcentage en poids du polymère entérique soluble dans l'eau est compris entre 3 % en poids et 25 % en poids, le pourcentage en poids du polymère filmogène soluble dans l'eau est compris entre 71 % en poids et 96,5 % en poids, le pourcentage en poids du coagulant est compris entre 0,5 % en poids et 3 % en poids, et le pourcentage en poids de l'auxiliaire de gélification est compris entre 0,005 % en poids et 1 % en poids,
dans laquelle un rapport des groupes fonctionnels hydrophobes aux groupes fonctionnels hydrophiles dudit polymère entérique soluble dans l'eau est compris entre 30 : 70 et 40 : 60, et
dans laquelle le polymère entérique soluble dans l'eau présente un poids moléculaire de 20 kDa à 1000 kDa inclusivement, le polymère filmogène soluble dans l'eau présente un poids moléculaire de 50 kDa à 815 kDa inclusivement, et le coagulant présente un poids moléculaire de 450 kDa à 550 kDa inclusivement.

2. Composition d'enveloppe de capsule résistante aux acides selon la revendication 1, dans laquelle les groupes fonctionnels hydrophobes dudit polymère entérique soluble dans l'eau sont choisis parmi un groupe méthoxy, un propylène glycol, un groupe méthyle, un groupe éthyle, ou une combinaison de ceux-ci, de préférence un groupe méthoxy.

3. Composition d'enveloppe de capsule résistante aux acides selon l'une quelconque des revendications 1 à 2, dans laquelle les groupes fonctionnels hydrophiles dudit polymère entérique soluble dans l'eau comprennent un groupe carboxy, un groupe amide, un groupe hydroxy, ou une combinaison de ceux-ci.

4. Composition d'enveloppe de capsule résistante aux acides selon l'une quelconque des revendications 1 à 3, dans laquelle les groupes fonctionnels hydrophiles dudit polymère entérique soluble dans l'eau comprennent un groupe carboxy.

5. Composition d'enveloppe de capsule résistante aux acides selon l'une quelconque des revendications 1 à 4, dans laquelle ledit polymère entérique soluble dans l'eau comprend au moins l'un choisi parmi les groupes suivants : pectine, alginate de propylène glycol, et gomme xanthane.

6. Composition d'enveloppe de capsule résistante aux acides selon l'une quelconque des revendications 1 à 5, dans laquelle ledit polymère entérique soluble dans l'eau comprend la pectine ou l'alginate de propylène glycol.

7. Procédé pour préparer une enveloppe de capsule résistante aux acides, comprenant les étapes consistant à :
dissoudre la composition d'enveloppe de capsule résistante aux acides selon l'une quelconque des revendications 1 à 6 dans de l'eau désionisée pour former une solution d'enveloppe de capsule,
chauffer et ensuite laisser refroidir la solution d'enveloppe de capsule pour former une solution mère d'enveloppe de capsule,
immerger une tige dans la solution mère d'enveloppe de capsule, puis retirer la tige pour former une tige revêtue de couche mince, et
sécher ladite tige revêtue de couche mince pour former ladite enveloppe de capsule résistante aux acides.

8. Procédé pour préparer une enveloppe de capsule résistante aux acides selon la revendication 7, dans lequel l'étape consistant à chauffer et ensuite laisser refroidir la solution d'enveloppe de capsule pour former ladite solution mère d'enveloppe de capsule comprend en outre l'étape consistant à chauffer la solution d'enveloppe de capsule à une température comprise entre 65°C et 90°C, et ensuite l'étape consistant à laisser refroidir la solution d'enveloppe de capsule pour former la solution mère d'enveloppe de capsule.

9. Procédé pour préparer une enveloppe de capsule résistante aux acides selon la revendication 7 ou 8, dans lequel l'étape consistant à sécher la tige revêtue de couche mince pour former ladite enveloppe de capsule résistante aux acides comprend en outre l'étape consistant à sécher la tige revêtue de couche mince à une température comprise entre 20°C et 90°C pour former ladite enveloppe de capsule résistante aux acides.
